# EUROPÄISCHE PATENTSCHRIFT

(11) **EP 0 459 388 B1**
(45) Veröffentlichungstag und Bekanntmachung des Hinweises auf die Patenterteilung: **18.01.1995**
(21) Anmeldenummer: 91108675.9
(22) Anmeldetag: 27.05.1991
(51) Int. Cl.: A61B 5/16

(54) **Vorrichtung zur Erzeugung von Tastreizen durch Vibration eines auf die Haut eines Menschen aufzusetzenden Stössels**
Device for generation of tactile stimuli by vibration of a plunger placed on the human skin
Dispositif générateur de stimulations tactiles par vibration d'un piston placé sur la peau humaine

(30) Priorität: 29.05.1990 DE 4017251
(43) Veröffentlichungstag der Anmeldung: 04.12.1991
(73) Patentinhaber: Erich Jaeger GmbH & Co. KG, D-97204 Höchberg (DE)
(72) Erfinder: Schumacher, Walter, W-3344 Ohrum (DE); Wetzel, Walter, W-3300 Braunschweig (DE); Hollnagel, Gerd, W-3300 Braunschweig (DE); Arndt, Gerald, W-3300 Braunschweig (DE); Plüquett, Ulrich, W-3400 Göttingen-Nikolausberg (DE); May, Arno, W-3400 Göttingen (DE); Oberdorfer, Dietmar, W-3400 Göttingen (DE); Baden, Heiko, W-3043 Schneverdingen 4 (DE)
(74) Vertreter: Bauer, Wulf, Dr.

(56) Entgegenhaltungen:
- EP-A- 0 276 407
- AT-B- 379 898
- DD-A- 160 854
- DD-A- 227 588
- SOVIET INVENTIONS ILLUSTRATED Woche 9010, 18. April 1990,Zusammenfassung Nr. 90-073535 P/31, Derwent Publications Ltd., London, GB;& SU - A - 1503745 (KIEV WORK DISEASES) 30.08.1989

## Beschreibung

Die Erfindung bezieht sich auf eine Vorrichtung zur Erzeugung von Tastreizen durch Vibration eines auf die Haut eines Menschen aufzusetzenden Stößels. Die Vorrichtung hat einen Meßkopf, der einerseits ein Gehäuse aufweist, an dem der hin- und herbewegbare Stößel angeordnet ist und der andererseits eine Antriebsvorrichtung für den Stößel hat.

Mit derartigen, z.B. aus den Druckschriften DD-A-227 588 und DD-A-160 854 bekannten Vorrichtungen kann die Vibrationswahrnehmung erfaßt werden, so kann beispielsweise bei ansteigender oder abfallender Vibrationsamplitude ein Schwellenwert für die Wahrnehmung der Vibration durch einen Patienten angegeben werden. Hieraus wiederum lassen sich Aussagen über die Reizleitfähigkeit der großkalibrigen Nervenfasern (des Tastsinns) machen. Durch Vergleich mit Normdaten gesunder Patienten oder den Daten desselben Patienten aus früheren Messungen und unter gleichen Bedingungen kann eine Frühdiagnose bei sensiblen Polyneuropathien, z. B. in Zusammenhang mit Diabetes mellitus, Nierenerkrankungen, toxischen Nervenschädigungen, multipler Sklerose und Rückenmarkserkrankungen erfolgen.

Aus der AT-A-379898 ist eine Vorrichtung mit einem an einem Stößel angebrachten Beschleunigungssensor bekannt, welche jedoch lediglich zur Aufnahme von Schwingungen eines Bauteils verwendet werden kann.

Bei der vorbekannten Einrichtung der eingangs genannten Art ist die Amplitude der Schwingung des Stößels einstellbar. Die Amplitude wird durch eine Lichtschranke als Auslenkung gegenüber der Hauptmasse des Meßkopfes erfaßt.

Bei der vorbekannten Vorrichtung ist die Amplitude jedoch in starkem Maße von der Kraft, mit der der Stößel auf die Haut aufgedrückt wird, bzw. einer externen Dämpfung des Stößels abhängig. Insbesondere bei kleinen Amplituden ist es dadurch schwierig, eine sichere Ablesung und damit Bestimmung der Amplitude zu erhalten. Der Amplitudenwert ist nicht ausreichend reproduzierbar. Schließlich wird die Auflagekraft nur in wenigen, groben Stufen angezeigt, auch dies erschwert eine Reproduzierbarkeit einer Messung mit der vorbekannten Vorrichtung.

Ausgehend von der Vorrichtung der eingangs genannten Art hat sich die Erfindung die Aufgabe gestellt, diese Vorrichtung dahingehend weiterzuentwickeln, daß unabhängig von der Andrückkraft auf die Haut eines Menschen und unabhängig von einer eventuellen äußeren Dämpfung die Amplitude der Schwingung des Stößels exakt und damit reproduzierbar vorgegeben und eingehalten werden kann. Damit ist sie unabhängig von der Auflagekraft. Auch die Auflagekraft soll in einer Weiterbildung präzise erfaßt werden können, so daß auch hierdurch die Reproduzierbarkeit einer Messung verbessert wird.

Diese Aufgabe wird durch eine Vorrichtung mit den Merkmalen des Patentanspruchs 1 gelöst.

Erfindungsgemäß wird somit die Beschleunigung des Stößels in jedem Zeitpunkt seiner periodischen Hin- und Herbewegung erfaßt. Das erhaltene Beschleunigungssignal wird zur Steuerung des Antriebs des Stößels eingesetzt. Auf diese Weise läßt sich die Schwingungsamplitude präzise vorbestimmen und unabhängig von der Auflagekraft oder sonstiger Dämpfung einhalten. Weiterhin kann aus dem Beschleunigungssignal durch Integration ein Geschwindigkeitssignal und durch weitere Integrationen ein Wegsignal erhalten werden.

Die den Stößel bewegende Antriebsvorrichtung und/oder der Beschleunigungssensor sind in einer vorteilhaften Weiterbildung elektrodynamisch ausgeführt, also weisen jeweils einen Permanentmagneten und eine Spule auf, die Spule befindet sich im Feld des Permanentmagneten. Eine derartige Antriebsvorrichtung wirkt in beide Antriebsrichtungen, hierdurch wird im Gegensatz zu in nur einer Richtung arbeitenden Antriebsvorrichtungen, die einer rückstellenden Feder bedürfen, eine gleichmäßigere Vibrationsbewegung erzielt. Auch der Beschleunigungssensor dieser Art gibt in beiden Bewegungsrichtungen des Stößels ein Signal ab, wobei sich die Signale noch durch das Vorzeichen unterscheiden. Dadurch läßt sich überprüfen, ob die Hinbewegung tatsächlich mit der Rückbewegung identisch ist und lassen sich Abweichungen in den beiden Bewegungsrichtungen über einen Regelverstärker ausgleichen.

In einer anderen Ausführung ist der Beschleunigungssensor piezoelektrisch ausgeführt. Diese Ausbildung hat den Vorteil einer sehr robusten, einfachen Konstruktion. Der Sensor muß ausschließlich mit dem Stößel verbunden werden und hat keine Teile, die am Gehäuse zu befestigen sind, demgemäß entfällt eine präzise Führung zwischen Stößel und Gehäuse, ein Luftspalt wie bei einer elektrodynamischen Ausbildung muß nicht präzise eingehalten werden usw.

In einer weiteren, vorteilhaften Ausbildung ist der Beschleunigungssensor an einen Integrator angeschlossen, dessen Ausgang mit der Antriebsvorrichtung verbunden ist. Hierdurch wird eine Regelung der Vibrationsbewegung des Stößels auf konstante Amplitude erzielt, sofern eine Gegenkopplung vorliegt. Wird die Amplitude der Bewegungen des Stößels größer, so wird auch das vom Beschleunigungssensor erfaßte Signal größer, dieses wiederum wird nach Integration dazu benutzt, den Antrieb des Stößels zu verringern.

Schaltet man in einer anderen, bevorzugten weiterbildung dem Beschleunigungssensor einen zweifachen Integrator nach, so erhält man eine Angabe über den Weg des Stößels im Verlauf der Zeit.

In einer vorteilhaften Weiterbildung ist der Stößel mit dem Gehäuse durch Federn verbunden, wobei die Federn einerseits die Andrückkraft des Stößels bestimmen und andererseits in vorteilhafter Weiterbildung den Stößel im Gehäuse axial verschiebbar halten. Hierzu sind mindestens zwei axial versetzt angeordnete Blattfedern in einer bevorzugten Ausführung vorgesehen.

In einer weiteren vorteilhaften Ausführung ist der mindestens einen Feder, die den Stößel gegenüber dem Gehäuse abstützt, ein Sensor für das Erfassen der Auslenkung des Stößels gegenüber dem Gehäuse zugeordnet. Über diesen Sensor und die Federkonstante der mindestens einen Feder läßt sich die Kraft erfassen, mit der der Stößel in das Gehäuse eingedrückt (oder aus ihm herausgezogen) wird.

Weiterhin ist es vorteilhaft, die Frequenz der Bewegungen des Stößels einstellbar zu halten, also insbesondere den Antrieb mit einem Generator einstellbarer Frequenz zu verbinden. Dadurch können Tastreize unterschiedlicher Schwingungsfrequenzen erzeugt und es kann zusätzlich die Abhängigkeit der Wahrnehmung eines Patienten von der Frequenz der Bewegungen des Stößels untersucht werden.

Der Vibrator ist schließlich in einer Weiterbildung an einem einstellbaren Gelenkarm lösbar befestigt. Er kann so an unterschiedlichen Partien eines Patienten angesetzt werden. Wird er vom Gelenkarm gelöst, kann er auch manuell eingesetzt werden.

Weitere Vorteile und Merkmale der Erfindung ergeben sich aus den übrigen Ansprüchen sowie der nun folgenden Beschreibung eines nicht einschränkend zu verstehenden Ausführungsbeispiels, das unter Bezugnahme auf die Zeichnung näher erläutert wird. In dieser zeigen:
- Fig. 1: eine Prinzipskizze in Blockausführung der gesamten Vorrichtung und
- Fig. 2: ein Schnittbild durch einen Meßkopf.

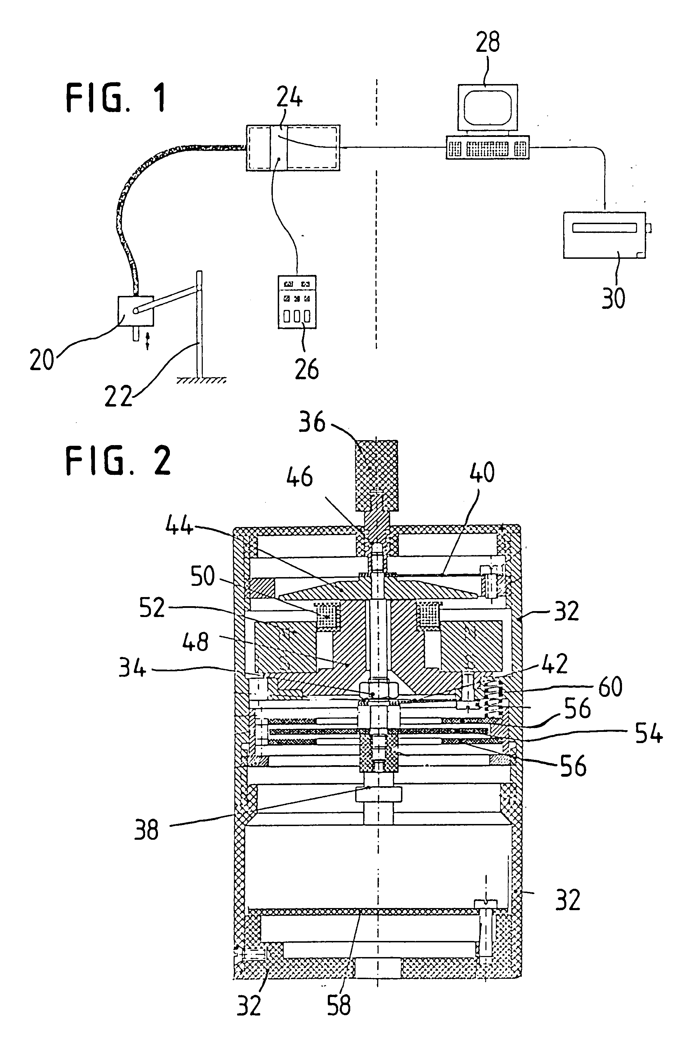

Die Vorrichtung gemäß Fig. 1, auch Vibrationsreizgerät genannt, besteht aus den Baugruppen Meßkopf 20, Meßkopfhalterung 22, Steuerelektronik 24, Patientenbedienfeld 26 und Personal-Computer 28 mit angeschlossenem Drucker 30.

Auf den Meßkopf 30 wird im folgenden einzeln eingegangen. Die Verbindung zwischen Meßkopf 20 und Steuerelektronik 24 erfolgt über eine flexible, mechanisch geschützte Zuleitung. Die Meßkopfhalterung 22 ist an einem Stativ oder einer ähnlichen Halterung befestigt, sie hat die Aufgabe, den Meßkopf in unterschiedlichen Winkelstellungen und unterschiedlichen Orten so zu halten, daß er auf beliebige Hautpartien eines Patienten aufgesetzt und in dieser Position gehalten werden kann.

In der Steuerelektronik 24 findet die Signalverarbeitung der Sensoren des Meßkopfes statt, weiterhin befinden sich in ihr die elektrischen Schaltungen der Antriebsvorrichtung für den Meßkopf.

Das Patienten-Bedienfeld 26 hat fünf Leuchtfelder und drei Taster, letztere sind farblich gekennzeichnet. Weiterhin hat es Piezosummer zur Erzeugung von Zwei Signaltönen. Über das Patienten-Bedienfeld 26 gibt ein Patient ein, ob er einen Tastreiz wahrnimmt oder nicht. Die jeweils für das einzelne Untersuchungsprogramm zu drückende Taste wird durch Lichtsignal angezeigt.

Der Meßkopf 20 hat ein im wesentlichen zylindrisches Gehäuse in dem axial ein hin- und herbewegbarer Stößel 34 angeordnet ist, der aus einer Stirnseite des Gehäuses herausragt und dort in einem Stößelkopf 36 endet. An seinem anderen, innerhalb des Gehäuses 32 befindlichen Ende ist ein piezoelektrischer Beschleunigungssensor 38 (Typ PCB 303 A11) angeordnet.

Der Stößel 34 ist durch eine obere Blattfeder 40 und durch eine untere Blattfeder 42 einerseits im Gehäuse 32 axial geführt und andererseits gegenüber dem Gehäuse 32 elastisch angeordnet.

Der Stößelkopf 36 ist ein Kunststoffteil, er hat einen Durchmesser von 10 mm. Er ist auswechselbar, kann also bei Beschädigungen oder zur Reinigung (Sterilisierung) vom eigentlichen Stößel 34 abgeschraubt werden.

Zwischen den beiden Blattfedern 40, 42, die in einem möglichst großen Abstand voneinander angeordnet sind, befindet sich der elektrodynamische Antrieb des Stößels 34. Hierzu ist der Stößel 34 mit einer scheibenförmigen Polplatte 44 aus ferromagnetischem Material verbunden, sie ist zwischen einem oberen, mit dem Stößelkopf 36 verbundenen Teilstück 46 aus Kunststoff des Stößels 34 und einer Schulter des bolzenförmigen Hauptstücks des Stößels 34 eingespannt. Unmittelbar unterhalb der Polplatte 44 und durch einen Luftspalt einer Breite von etwa 0,1 mm von ihr getrennt befindet sich ein Polkern 48, der ebenfalls aus ferromagnetischem Material gefertigt ist. Er wird von einer Spule 50 umgriffen, die sich in unmittelbarer Nähe der Polplatte 44 befindet. Außerhalb und unterhalb dieser Spule 50 ist ein ringförmiger Magnet angeordnet, die Polarität ist angegeben.

Durch Erregen der Spule 50 mit einem Wechselstrom wird eine hin- und hergehende Bewegung der Polplatte 44 und damit des Stößels 34 erreicht. Je nach Ansteuerung der Spule 50 können Amplituden zwischen 0 und 0,15 mm eingestellt werden. Das Schwingungsverhalten ist sinusförmig. Die Spule 50 ist mit einem in der Steuerelektronik 24 angeordneten Oszillator einstellbarer Frequenz verbunden, ihm ist ein Leistungsverstärker nachgeschaltet, der ebenfalls in der Steuerelektronik 24 angeordnet ist und ausgangsseitig mit der Spule 50 verbunden ist.

Unterhalb der unteren Blattfeder 42 und in ihrer unmittelbaren Nähe befindet sich eine Kondensatoranordnung, mit deren Hilfe der Weg erfaßt werden kann, mit der die beiden Feder 40, 42 und somit der Stößel 34 aus der Ruhestellung (entspannte Federn 40, 42) ausgelenkt ist. Wird der Stößelkopf 36 auf die Haut eines Patienten aufgesetzt, so erfolgt dies mit einem gewissen Auflagedruck, also einer Auslenkung der beiden Blattfedern 40, 42. Das Maß dieser Auslenkung wird durch die Kondensatoranordnung bestimmt. Die Auslenkung kann (in beiden Richtungen) ca. 1 mm betragen. Die jeweils aktuell vorliegende Auflagekraft wird auf dem Bildschirm des Computers 28 durch einen Balken angezeigt.

Am Stößel 34 ist eine scheibenförmige, mittlere Sensorplatte 54 befestigt, die sich zwischen zwei mit dem Gehäuse 32 verbundenen, ringförmigen Sensorplatinen 56 gleicher Ausbildung befindet. In der gezeigten Ruhestellung ist der Abstand der mittleren Sensorplatte 54 von den beiden Sensorplatten 56 gleich groß. Wird die mittlere Sensorplatte 54 durch Verschieben des Stößels 34 gegenüber dieser Ruhelage ausgelenkt, so ändert sich die Kapazität der Anordnung, die Kapazitätsänderung wird als Maß für die Wegänderung benutzt.

Die beiden Sensorplatinen 52 werden in Ruhestellung des Stößels so justiert, daß sie allseitig gleichen Abstand von der mittleren Sensorplatte 54 hat. Die Justierung wird durch eine Justierfeder 60 erleichtert.

Im unteren Innenraum des Gehäuses 32 ist eine Platine 58 für die Aufnahme elektronischer Bauelemente für die Kondensatoranordnung vorgesehen.

## Patentansprüche

1. Vorrichtung zur Erzeugung von Tastreizen durch Vibration eines auf die Haut eines Menschen aufzusetzenden Stößels (34), mit einem Meßkopf (20), der einerseits ein Gehäuse (32) aufweist, in dem sich der hin- und herbewegbare Stößel (34) befindet und der andererseits eine Antriebsvorrichtung für den Stößel (34) aufweist,
dadurch gekennzeichnet, daß am Stößel (34) ein Beschleunigungssensor (38) angeordnet ist, und daß das Signal aus dem Beschleunigungssensor (38) zur Steuerung der Antriebsvorrichtung des Stößels (34) verwendet wird.

2. Vorrichtung nach Anspruch 1, dadurch gekennzeichnet, daß der Stößel (34) durch elastische Mittel, insbesondere Blattfedern (40, 42) im Gehäuse (32) elastisch gehalten und geführt ist.

3. Vorrichtung nach Anspruch 2, dadurch gekennzeichnet, daß zwischen Stößel (34) und Gehäuse (32) ein Weggeber angeordnet ist, der vorzugsweise als Kondensatoranordnung (54, 56) ausgeführt ist.

4. Vorrichtung nach Anspruch 1, dadurch gekennzeichnet, daß die Antriebsvorrichtung des Stößels (34) elektrodynamisch ausgeführt ist, daß insbesondere am Stößel (34) eine scheibenförmige Polplatte (44) befestigt ist, der eine Spule (50), ein Polkern (48) und ein Magnet (52), die jeweils mit dem Gehäuse verbunden sind, gegenüberstehen.

5. Vorrichtung nach Anspruch 1, dadurch gekennzeichnet, daß der Beschleunigungssensor (38) piezoelektrisch oder elektrodynamisch ausgebildet ist.

6. Vorrichtung nach Anspruch 1, dadurch gekennzeichnet, daß dem Beschleunigungssensor (38) ein Integrator nachgeschaltet ist, dessen Ausgang mit der Antriebsvorrichtung des Stößels (34) verbunden ist.

7. Vorrichtung nach Anspruch 1, dadurch gekennzeichnet, daß die Antriebsvorrichtung einen Generator einstellbarer Frequenz aufweist.

8. Vorrichtung nach Anspruch 1, dadurch gekennzeichnet, daß sie eine Anzeigevorrichtung für das Maß der Auflagekraft des Stößels (34) auf die Haut eines Patienten aufweist.

9. Vorrichtung nach Anspruch 1, gekennzeichnet durch ein bewegliches, handliches Patienten-Bedienfeld (26), an dem der Patient seine Reaktionen eingibt.

10. Vorrichtung nach Anspruch 1, dadurch gekennzeichnet, daß der Meßkopf (20) an einem einstellbaren Gelenkarm (Meßkopfhalterung 22) lösbar befestigt ist.

## Claims

1. Device for generation of tactile stimuli by vibration of a plunger (34) placed on the human skin, which device has a measuring head (20) comprising on one hand a housing (32), in which the reciprocating plunger (34) is arranged, and on the other hand an actuation unit for the plunger (34), characterized in that the accelaration sensor (38) is arranged at the plunger (34) and that the signal of this acceleration sensor (38) is used to control the actuation unit of the plunger (34).

2. Device according to claim 1, characterized in that the plunger (34) is elastically held and guided in the housing (32) by elastic means, especially blade springs (40, 42).

3. Device according to claim 2, characterized in that a position sensor is arranged between the plunger (34) and the housing (32), which sensor preferentially is realized by a condensor arrangement (54, 56).

4. Deviche according to claim 1, characterized in that the actuating unit of the plunger (34) is electrodynamic and in that a disk-shaped pole plate (44) is fixed especially to the plunger (34) and coopera tes with a coil (40), a pole core (48) and a permanent magnet (52), which are fixed to the housing (32), respectively.

5. Device according to claim 1, characterized in that the acceleration sensor (38) is piezoelectric or electrodynamic.

6. Device according to claim 1, characterized in that an integrator follows the acceleration sensor (38), the output thereof being connected to the actuating unit of the plunger (34).

7. Device according to claim 1, characterized in that the actuating unit comprises a generator whose frequency is adjustable.

8. Device according to claim 1, characterized by means for displaying a value of the force of the plunger (34) on the skin of a patient.

9. Device according to claim 1, characterized by a moveable, handy patient reaction panel (26), on which a patient feeds in his or her reactions.

10. Device according to claim 1, characterized in that the measuring head (20) is releasably fixed to an adjustable swivel arm (measuring head holder 22).

## Revendications

1. Dispositif générateur de stimulations tactiles par vibration d'un piston placé sur la peau humaine avec une tête de mesure (20) comprenant d'une part un boitier (32), dans lequel le piston (34) mobile de côté à l'autre est arrangé et d'autre part un mécanisme de commande pour le piston (34), characterisé en ce qu'un capteur d'accélération est arrangé au piston (34) et en ce que le signal de ce capteur d'accélération est utilisé pour le contrôle du mécanisme de commande du piston (34).

2. Dispositif selon la revendication 1, characterisé en ce que le piston (34) est fixé elastiquement et guidé par de moyens élastiques, espécialement de ressort de lame.

3. Dispositif selon la revendication 2, characterisé en ce que qu'un palpeur de position est arrangé entre le piston (34) et le boitier (32), quel palpeur est préférablement un arrangement des condensateurs (54, 56).

4. Dispositif selon la revendication 1, characterisé en ce que le mécanisme de commande du piston (34) est formé d'une facon électrodynamique et en ce que une plaque polaire en forme de disque est arrangé préférablement au piston (34), quelle plaque polaire est opposée d'une bobine (50), d'un noyeau polaire et d'un aimant (52), qui en chaque cas sont reliés avec le boitier.

5. Dispositif selon la revendication 1, characterisé en ce que le capteur d'accélération est piezoélectrique ou électrodynamique.

6. Dispositif selon la revendication 1, characterisé en ce que le capteur d'accélération (38) est suivi d'un intégrateur, la sortie duquel est relié avec le mécanisme de commande du piston (34).

7. Dispositif selon la revendication 1, characterisé en ce que le mécanisme de commande comprend un générateur à fréquence réglable.

8. Dispositif selon la revendication 1, characterisé en ce qu'il comport un tableau de signalation pour la valeur de la force d'appui du piston (34) sur la peau d'un client.

9. Dispositif selon la revendication 1, characterisé par un pupitre de commande (26) amovible et maniable du client, avec lequel le client présent ses réactions.

10. Dispositif selon la revendication 1, characterisé en ce que la tête de mesure est fixée à un bras articulé (fixation de la tête de mésure 22) d'une maniére amovible.
